(19) Europäisches Patentamt
European Patent Office
Office européen des brevets

(11) Publication number: **0 410 637 A1**

(12) **EUROPEAN PATENT APPLICATION**

(21) Application number: **90307857.4**

(22) Date of filing: **18.07.90**

(51) Int. Cl.[5]: **G01N 33/573**, G01N 33/543, C07K 3/02

(30) Priority: **19.07.89 IE 2333/89**

(43) Date of publication of application: **30.01.91 Bulletin 91/05**

(84) Designated Contracting States: **AT BE CH DE DK ES FR GB GR IT LI LU NL SE**

(71) Applicant: **BIORESEARCH IRELAND**
**a division of EOLAS, the Irish Science and Technology Agency, Glasnevin**
**Dublin 9(IE)**

Applicant: **Headon, Denis Robert**
**Aughanacurra**
**Dangan, Galway(IE)**

Applicant: **McSweeney, Finbarr Mary**
**Glencullen House**
**Glencullen, County Dublin(IE)**

Applicant: **UNIVERSITY COLLEGE GALWAY**
**The National University of Ireland**
**Galway(IE)**

(72) Inventor: **Headon, Denis Robert**
**Aughanacurra**
**Dangan, Galway(IE)**
Inventor: **McSweeney, Finbarr Mary**
**Glencullen House**
**Glencullen, County Dublin(IE)**

(74) Representative: **Caro, William Egerton et al**
**J. MILLER & CO. Lincoln House 296-302 High Holbornorn**
**London WC1V 7JH(GB)**

(54) **Diagnostic assays and their use in the assessment of clinical conditions.**

(57) Diagnostic assays for the detection and determination of either component of the Δ-6 desaturase antigen-antibody reaction are described which are useful in the assessment of a wide range of clinical conditions, including atopic eczema and premenstrual syndrome, wherein reduced levels of metabolites of linoleic acid may be attributable to a partial deficiency of the Δ-6 desaturase enzyme. The assays include various immunoassays.

EP 0 410 637 A1

This invention relates to diagnostic assays and to their use in the assessment of a variety of clinical conditions. More particularly, the invention relates to an immunoassay for use in the detection of an enzyme which may be indicative of a deficiency state in an individual.

Δ-6 desaturase is a membrane-bound enzyme which converts essential fatty acids in the diet to metabolic intermediates, further metabolites of which are involved in the tissue immune response, coagulation and vascular tone. If present in adequate amounts, the enzyme converts the essential fatty acid cis-linoleic acid to gamma-linolenic acid. Partial deficiencies of Δ-6 desaturase are considered to cause or increase liability to many chronic conditions in the general population. Conditions associated with inadequate levels of Δ-6 desaturase include atopic dermatitis and disorders associated with atopy, premenstrual syndrome, breast pain (mastalgia), Raynaud's phenomenon, rheumatoid arthritis, hypertension, coronary heart disease, vascular thrombosis, cancer, alcoholism, stress, high cholesterol levels, viral diseases and diabetes. Clinical studies (Brush, M.G. et al. (1984) Am. J. Obstet. Gynecol. 15 No. 4, pp 363-366) have shown that the levels of linoleic acid were significantly above normal and concentration of all metabolites of linoleic acids were significantly reduced in plasma of women suffering from premenstrual syndrome (PMS). These phenomena suggest a defect in the conversion of linoleic acid to gamma-linolenic acid. This deficiency is helieved to sensitize tissues so that they respond abnormally to normal levels of reproductive hormones.

Brush, M.G. (Omega-6 Essential Fatty Acids: Pathophysiology and Roles in Clinical Medicine pp 513-522 (1990) Alan R. Liss, Inc.) reports that there is a significant partial block of Δ-6 desaturase th PMS sufferers leading to impaired biosynthesis of n-6 essential fatty acids (EFAs) . This may be bypassed by gamma-linolenic acid treatment. It is postulated that the reduced levels of metabolites of linoleic acid are due to a partial deficiency of Δ-6 desaturase. The addition of various co-factors such as vitamin B6 and zinc are found to alleviate the condition in some sufferers as such co-factors are necessary for optimal functioning of the n-6 biosynthetic pathway.

Administration of gamma-linolenic acid, especially in the form of evening primrose oil, is currently gathering popularity as a treatment for premenstrual syndrome and is found to alleviate the symptoms of those suffering therefrom. However, as yet there is no convenient and certain diagnostic test as a basis for such treatment with evening primrose oil. Evening primrose oil is the naturally occurring seed oil of the evening primrose plant ( Oenothera biennis ) which contains high amounts of polyunsaturated fatty acids, 9% by weight of gamma-linolenic acid in the form of triglycerides. A particular form of evening primrose oil sold under the Trade Mark EPOGAM has been used by Morse, P.F. et al (British Journal of Dermatology (1989) **121**, 75-90) in a number of controlled trials for the treatment of atopic eczema. The results of these trials support studies which suggest that atopic eczema may be related to essential fatty acid metabolism, specifically significantly reduced levels of metabolites of linoleic acid viz. gamma-linolenic acid, dihomogammalinolenic acid and arachidonic acid.

EPOGAM is the seed oil from strains of the evening primrose plant selected and bred to yield oil of constant composition. The findings of Brush, M.G. (1990) supra are confirmed by O'Brien, P.M.S. and Massil, H. (Omega-6 Essential Fatty Acids: Pathophysiology and Roles in Clinical Medicine pp 523-545 (1990) Alan R. Liss, Inc. ).

Accordingly, there is a need for an assay, especially a blood-based assay, which could be used inter alia to assay blood samples over the course of the female menstrual cycle to provide an indication of changes in Δ-6 desaturase levels which would provide a firm clinical basis for evening primrose oil therapy in conditions such as premenstrual syndrome in women, but also in clinical medicine generally, especially in the conditions mentioned above.

In liver Δ-6 desaturase is found associated with the microsome fraction of liver cells. In animals, the enzyme Δ-6 desaturase, when present, is found to be homologous, such that, for example, essentially the same enzyme activity is found in both humans and the rat. Accordingly, a rat liver can be used as a ready source of material for the study of this enzyme and the results extrapolated to the human situation. The risks of obtaining samples of liver from human cadavers as a source of material such as antibodies, enzymes, etc. are well known and can be potentially hazardous when the liver is infected, for example, with hepatitis, especially virulent forms of heptatitis such as δ-heptatitis.

The enzyme Δ-6 desaturase requires NADH or NADPH and molecular oxygen as co-factors. In rat liver microsomes, Δ-6 desaturase converts linoleoyl-CoA to gamma-linolenoyl-CoA in the presence of the aforementioned co-factors according to Scheme 1.

Scheme 1

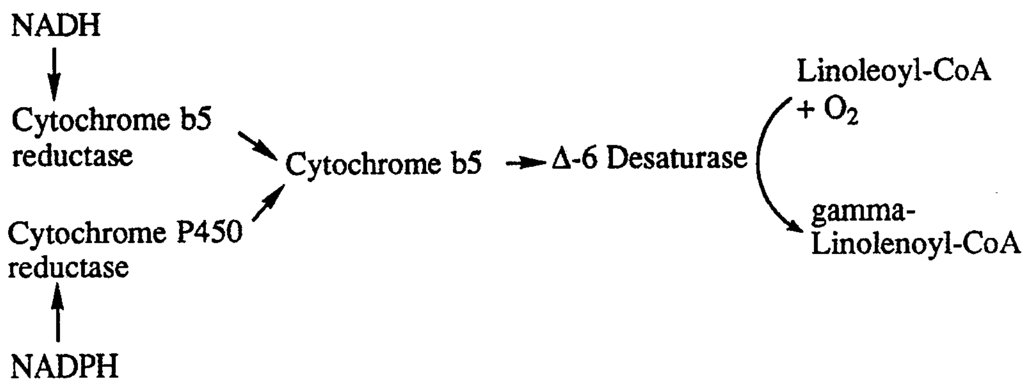


******

Δ-6 desaturase activity in blood/serum has not been detected to date.

The invention provides in one aspect a diagnostic method for the detection and determination of either component of the Δ-6 desaturase antigen-antibody reaction, which method comprises contacting a sample of a body fluid or secretion containing or suspected of containing the component to be detected with an amount of said other component and determining whether binding of said components has occurred.

The binding of the components may be estimated by Western Blotting in a manner known per se . The invention also provides an immunoassay method for the detection and determination of either component of the Δ-6 desaturase antigen-antibody reaction which comprises:

a) adding a sample of a body fluid or secretion containing or suspected of containing the component to be detected or determined to an amount of said other component of said Δ-6 desaturase antigen-antibody reaction in an insolubilised form;

b) allowing the immunochemical reaction to take place; and

c) determining the presence in said sample of said component by confirming the binding thereof to said insolubilised form of said one component as defined in step a). The invention further provides an immunoassay method for the detection and determination of the Δ-6 desaturase antigen component of the Δ-6 desaturase antigen-antibody reaction which comprises:

a*) adding a sample of a body fluid or secretion containing or suspected of containing Δ-6 desaturase to an amount of anti-Δ-6 desaturase in an insolubilised form;

b*) allowing the immunochemical reaction to take place; and

c*) estimating the amount of Δ-6 desaturase bound to said insolubilised anti-Δ-6 desaturase. The immunoassay method for the detection and determination of Δ-6 desaturase may comprise

a**) adding a sample of a body fluid or secretion containing or suspected of containing Δ-6 desaturase to an amount of anti-Δ-6 desaturase in an insolubilised form;

b**) allowing the immunochemical reaction to take place, while simultaneously or subsequently adding a predetermined amount of labelled anti-Δ-6 desaturase; and

c**) estimating the amount of Δ-6 desaturase by determining the activity and/or amount of free or bound labelled anti-Δ-6 desaturase in a manner known per se.

In each of the above immunoassay methods the amount of bound Δ-6 desaturase is preferably determined enzymatically in a manner known per se.

The invention also provides an immunoassay method for the detection of anti-Δ-6 desaturase which comprises:

A) adding a sample of a body fluid or secretion containing or suspected of containing anti-Δ-6 desaturase to an amount of Δ-6 desaturase in an insolubilised form;

B) allowing the immunochemical reaction to take place; and

C) determining the presence in said sample of anti-Δ-6 desaturase by confirming the binding thereof to said insolubilised Δ-6 desaturase.

In the method according to the invention for the detection of anti-Δ-6 desaturase the amount of bound anti-Δ-6 desaturase is preferably determined enzymatically in a manner known per se.

Further, in the method according to the invention for the detection of anti-Δ-6 desaturase the bound anti-Δ-6 desaturase may be contacted with anti-mammalian immunoglobulin prior to the enzymatic determination.

In one application, the methods hereinabove specified for the detection of Δ-6 desaturase may be used for determining levels of Δ-6 desaturase throughout the menstrual cycle in females indicative of a predisposition to, or confirming the likelihood of, premenstrual syndrome in such females and which can be alleviated by a source of gamma-linolenic acid.

However, equally the methods hereinabove specified can be used in the diagnosis and screening of individuals in clinical medicine generally for the purpose of ascertaining a deficiency of Δ-6 desaturase which results in an impairement of the conversion of linoleic acid to gamma-linolenic acid with the consequences outlined above. For example, such assay methods could be used in assessing patients with atopic eczema as a basis for using evening primrose oil in the treatment of such patients (Morse, P.F., et al (1989) supra ).

The body fluid is preferably blood or a blood fraction, especially plasma or serum, or urine.

The insolubilised form of the component used in step a), a*) or a**) is suitably bound to a bead, membrane, particle, plate, rod, tube, well or the like of plastics material or glass in a manner known se. The insolubilised form of the component used in step a), a*) or a**) may also be bound to a surface of biological origin such as cells. The surface to which the insolubilised form of the component used in step a), a*) or a**) is bound will normally be a surface adapted for protein adsorption.

Most preferably, the surface will comprise a plastics microtitration plate or strip, latex bead or "dipstick" adapted for protein adsorption wherein or whereon the immunochemical reaction and the estimation of step c), c*) or c**) can take place. Especially suitable microtitration plates are gammairradiated microtitration plates. Examples of such gamma-irradiated microtitration plates are flat-well polystyrene microtitration plates marketed by Dynatech under the Trade Mark "MICROELISA" and those sold under the Trade Mark "NUNC" IMMULON.

Where the insolubilised component used in step a), a* or a**) is anti-Δ-6 desaturase, the relevant surface is preferably coated directly with an optimum dilution of polyclonal antibody prepared by separating the relevant immunoglobulin fraction of antiserum or, alternatively, monoclonal antibody.

Preferably, the estimation carried out in step c), c*) or c**) is carried out by enzymatic, fluorometric, luminometric or radiometric assay, using enzymes, fluorochromes, light-emitting probes or radio labels, respectively. The estimation carried out in step c), c*) or c**) may also involve the use of the Western Blotting technique.

The labelled agents for use in the aforementioned assays may be prepared in conventional manner or are purchased from appropriate suppliers. Such labelled agents are normally in the form of conjugates such as enzyme-labelled antibodies for use in competitive binding assays. The labelled agent may also suitably be an antibody covalently linked to a radiolabel for use in a radiometric assay. A suitable radiolabel is $^{125}I$.

Preferably, the estimation of the bound component in step c), c*) or c**) is carried out by enzyme immunoassay using a suitable peroxidase conjugate. A suitable peroxidase is horseradish peroxidase. Another suitable peroxidase conjugate is an avidin-biotin peroxidase complex, especially a streptavidin-biotin peroxidase complex which may be used with an antibody biotin conjugate to amplify the enzyme assay in conventional manner.

It will be appreciated by those skilled in the art that various types of assays may be employed in accordance with the invention for the detection of either component of the Δ-6 desaturase - anti-Δ-6 desaturase reaction. For example, appropriate anti-mammalian immunoglobulin, with or without a label, can be used to bind to anti-4-6 desaturase bound to an insolubilised form of Δ-6 desaturase as shown, for example, hereinafter in Example 6. It may also be appropriate in certain circumstances to use an anti-enzyme in an enzyme immunoassay for reacting with an enzyme-antibody conjugate.

The invention also provides test kits or packs containing the necessary components/ingredients for carrying out the methods according to the invention.

A test kit in accordance with the invention for the detection and determination of Δ-6 desaturase in a body fluid or secretion by immunoassay suitably comprises:

A′) a given quantity of insolubilised anti-Δ-6 desaturase or the necessary ingredients for preparing said insolubilised anti-Δ-6 desaturase; and

B′) a given quantity of the coupling product of a label with anti-Δ-6 desaturase.

When the immunoassay to be carried out is an enzyme immunoassay, component B′) will comprise a given quantity of the coupling product of an enzyme label with anti-Δ-6 desaturase. Such a test kit may suitably further comprise a substrate for the determination of the activity of said enzyme.

The antibody for use in accordance with the invention is an antibody capable of binding to one or more

epitope(s) on the enzyme Δ-6 desaturase. Thus the antibody for use in accordance with the invention may be a monoclonal antibody or polyclonal antibody. Such antibodies are preferably of the IgG class.

The monoclonal antibody or polyclonal antibody for use in accordance with the invention is preferably a mammalian monoclonal antibody or polyclonal antibody.

The monoclonal antibody may suitably be human, mouse or rat monoclonal antibody prepared by conventional methods, including recently developed methods for producing monoclonal antibody on a commercial scale, genetically engineered monoclonal antibody or antibody fragments or monoclonal antibody produced by in vitro immunisation of suitable cells.

Polyclonal antibody for use in accordance with the invention is suitably prepared by immunising a mammalian host with Δ-6 desaturase and recovering the antisera or anti-Δ-6 desaturase, as required.

In a further aspect of the invention there is provided a method for the production of polyclonal anti-Δ-6 desaturase which comprises immunising a mammalian host with Δ-6 desatrnrase bound to a carrier by injection intradermally or intraperitoneally, bleeding said host and collecting the antiserum.

In a preferred embodiment, the mammalian host is immunised with Δ-6 desaturase bound to fragments of a suitable gel which allows for a slow release of the antigen over a period of time following administration. A particularly suitable gel is polyacrylamide gel. The antigen in whatever form is administered intradermally (subcutaneously) or intraperitoneally.

A monoclonal antibody for use in accordance with the invention is preferably produced by a hybridoma formed by fusion of spleen cells from a human, mouse or rat previously immunised with Δ-6 desaturase and cells from a myeloma line selected from human, mouse and rat myeloma cell lines.

Mammalian monoclonal antibody in accordance with the invention may be prepared by a method which comprises:

i) immunising the mammal with Δ-6 desaturase;

ii) removing the spleen from said mammal and making a suspension of spleen cells;

iii) fusing said spleen cells with appropriate mammalian myeloma cells;

iv) screening the resultant hybridomas in separate wells, in a medium which will not support the unfused myeloma cells, for those with supernatants containing antibody which gives selective binding of Δ-6 desaturase;

v) selecting and cloning hybridomas producing the desired antibody; and

vi) recovering the antibody from the supernatant above said clones.

Alternatively, mammalian monoclonal antibody according to the invention may be prepared by a method which comprises preceding steps i)-v) followed by:

vi) transferring said clones intraperitoneally into a mammal; and

vii) harvesting the malignant ascites from said mammal.

In either of the above two methods, the mammals are suitably immunised with Δ-6 desaturase bound to fragments of a suitable gel. A particularly suitable gel is polyacrylamide gel.

In relation to the above methods, the animal systems used may be, for example, human-human, mouse-mouse, rat-mouse and rat-rat. An obvious exception would be a method in humans involving the transfer of clones intraperitoneally into a human and harvesting malignant ascites which would be considered unethical.

The invention will be further illustrated by the following Examples.

## EXAMPLE 1

### Extraction of rat liver microsomes

Microsomes were extracted from rat liver according to the following procedure which was carried out at 0-4 °C. The nucrosome extraction was carried out on ice (0 °C), except for the centrifugation steps which were carried out at 4 °C. A male rat 120-150 g was sacrificed and the liver rapidly excised and placed in 0.25 M sucrose to wash off the blood. After a quick washing, the liver was placed in a homogenisation buffer with the following composition:

0.25 M sucrose
5 mM EDTA
mM Tris-HCl pH 7.2

3 ml of buffer was used per gram of liver. A weight of 120-150 g is the normal weight range for healthy

male rats which are not on any diet. Selection of a male rat with a weight in this range prevents the selection of old rats with diminished enzyme activity, since enzyme activity decreases as age increases. The liver was homogenised in the buffer and then centrifuged for 30 minutes at 10,000 g (9,500 rpm.). The pellet obtained was discarded and the supernatant subjected to centrifugation at 27,000 (77,000 g av.) r.p.m. for 90 minutes. The microsome pellet thereby obtained was suspended in 0.1 M Tris-HCl at pH 7.2 and stored at -70 ° C.

## EXAMPLE 2

### Δ-6 Desaturase activity assay on rat liver microsomes

An assay for Δ-6 desaturase was carried out on the rat liver microsome preparation obtained in Example 1 according to Scheme 2.

Scheme 2

10mM NADH
5mM $Mg/Cl_2$
5mM ATP
0.1 mM CoA
2.94 nmol $^{14}$C-Linoleoyl-CoA (170 nCi)
97 nmol Linoleoyl-CoA

+ Microsome protein (2.5 mg)

↓ incubate at 37°C for 15 min. in phosphate buffer pH 7.2

Saponify and esterify the extracted free fatty acid.

↓

Measure the conversion of

[$^{14}$C] Linoleoyl acid to [$^{14}$C] gamma-Linolenoyl acid

by thin-layer chromatography of the fatty acid methyl esters on 4% $AgNO_3$ - impregnated silica gel plates and determine the radioactivity by scintillation counting.

******

The assay was carried out for different incubation times (5 min., min. and 30 min.) with different amounts of microsome protein (2.5 mg and 5 mg). As shown in Scheme 2 the conditions thereby selected for the assay were an rncubation time of 15 min., 2.5 mg microsome protein and an incubation temperature of 37° C. The Specific Activity of the enzyme was found to be 0.30 and the percentage conversion 12-13%. The total volume used for the enzyme reaction was 500 μl.

EXAMPLE 3

Δ-6 Desaturase assay on blood and plasma

10 ml of peripheral blood was taken by Vacutainer (Vacutainer is a Trade Mark) containing 143 usp$^{u}$ Li heparin and treated as depicted in Scheme 3.

Scheme 3

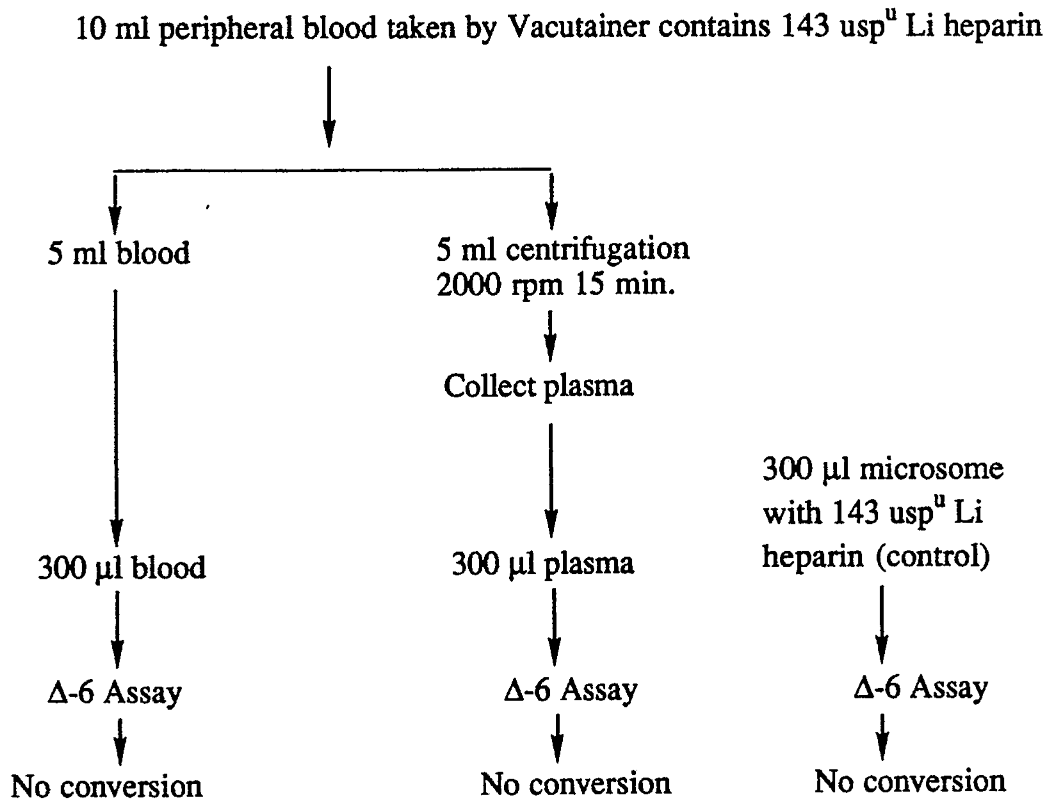


******

When the Δ-6 desaturase assay used in Example 2 was carried out on the blood and plasma samples no conversion of linoleoyl-CoA to gamma-linolenoyl-CoA was found. The control was included to determine whether the heparin had any effect on the assay. The results were consistent with one of the following situations:

1) The assay is not sensitive enough for the small amount of Δ-6 desaturase in blood/plasma.
2) There is no Δ-6 desaturase in blood/plasma.
3) There is a lack of co-factors for Δ-6 desaturase in blood/plasma.

Situation 3) was found to be the most plausible, since Δ-6 desaturase immunoactivity was detected in human serum.

The presence of Δ-6 desaturase in blood/plasma was also confirmed by the immunoassay method in accordance with the invention, specifically, by adding samples of blood and plasma to microtitre wells coated with anti-Δ-6 desaturase prepared in accordance with the invention and as described in Example 5.

## EXAMPLE 4

### Isolation of Δ-6 desaturase from rat liver microsomes

The microsomal fraction from rat liver homogenates prepared in Example 1 after differential centrifugation contains 15-30 mg/ml protein, from which Δ-6 desaturase was isolated following the procedure used by Okayasu, T. et al. (1981) (Archives of Biochemistry and Biophysics Vol. 206, No. 1, January pp 21-28) according to the procedure depicted in Scheme 4.

Scheme 4

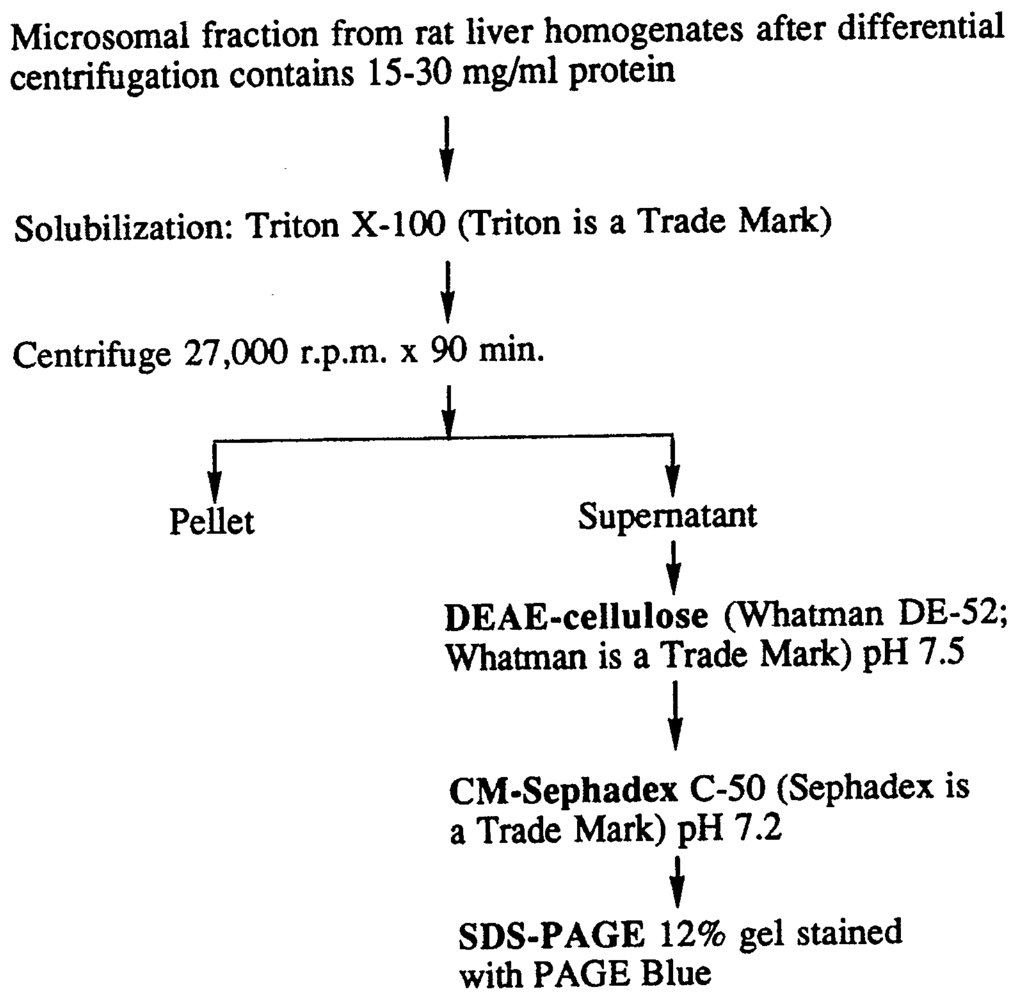


******

The results of the SDS-PAGE (sodium dodecylsulphate-polyacrylamide gel electrophoresis) using molecular weight markers resulted in a 66,000 molecular weight band in the lane corresponding to the microsomal fraction eluted from the CM-Sephadex C-50 column which was cut out and the Δ-6 desaturase isolated therefrom by electroelution. The Δ-6 desaturase fraction thereby obtained was stored for a brief period at -70 °C after concentration on a DIAFLO (DIAFLO is a Trade Mark) YM-10 ultra-filter (Amicon) and freeze-drying. A sample of the material obtained was checked by running it on 12% SDS-PAGE gel stained with silver for the presence of Δ-6 desaturase. The latter stain, which is sensitive at nanogram levels, is more sensitive than the PAGE Blue stain, which is sensitive at microgram levels, and which was used in the electrophoresis of the fraction obtained following treatment on CM-Sephadex C-50.

The 66,000 molecular weight band was also used to immunise rabbits to raise antibodies to Δ-6 desaturase as described in Example 5.

EXAMPLE 5

Production of polyclonal antibodies to Δ-6 desaturase

1.5 mg of material obtained from the CM-Sephadex C-50 column in Example 4 was run on 12% preparative PAGE stained with PAGE Blue. The 66 K band previously identified as being Δ-6 desaturase activity (Okayasu, T. et al. , (1981) supra was cut out and the gel was fragmented by passing it through an 18 gauge syringe in 2.0 ml of complete Freund's adjuvant. Two rabbits were unmunised by injecting them subcutaneously, each at at least 20 sites on the back, with a total of 1 ml of the Δ-6 desaturase-gel preparation. 28 days after immunisation the rabbits were boosted subcutaneously with the same amount of material prepared in the same way as for the immunisation step. Two weeks after boosting, the rabbits were bled (8-10 ml blood per rabbit) and the serum (10 ml) collected. Blood (20 ml) was also taken from a non-immunised rabbit and serum (7 ml) collected as a control. The production of antibodies was confirmed by enzyme linked immunosorbent assay (ELISA) as shown in Example 6 and by Western Blotting as shown in Examples 7 and 8.

EXAMPLE 6

ELISA for anti-Δ-6 desaturase

The 66,000 molecular weight band was excised from the gel and electroeluted. Material from a number of elutions was pooled and concentrated by ultrafiltration on a DIAFLO YM-10 ultra-filter and freeze dried. The concentrated material was stored at -70° C until required. To carry out ELISA, the material was resuspended in distilled water and diluted with coating buffer (0.05 M carbonate buffer, pH 9.6) to various dilutions (125-150 µg/ml) and coated onto microtitre plates. The plates were incubated for 1.5 h at 37° C and then washed with phosphate buffered saline (PBS). Antisera (1/1,000 dilution) as prepared in Example 5 was added to the plates which were incubated overnight at 4° C. Donkey anti-rabbit IgG conjugated to horseradish peroxidase (HRPO) was then added and the plates incubated for 1 hour at 37° C. The plates were then washed three times with a saline solution and dried by inverting and hitting on tissue placed on the bench surface. Phenylene diamine (OPD) was added as substrate and the colour reaction developed in the dark for 30 min. at room temperature. The reaction was stopped by the addition of 50 µl of 4 M $H_2SO_4$ and the plates were read at 492 nm. Bovine serum albumin (BSA) was used as a control and was coated on the microtitre plates in the same way as for the Δ-6 desaturase antigen. The results showed that the antisera prepared in Examples contains IgG antibody which specifically binds to the protein material electroeluted from the 66K band in Example 4.

EXAMPLE 7

Western Blot Assay Procedure for Δ-6 desaturase

Samples to be assayed for Δ-6 desaturase are first separated by SDS - polyacrylamide gel electrophoresis according to the method of Laemmli, U.K. (Nature (1970) 227 , 680-685). A 12% separating gel is prepared with a 4% stacking gel in accordance with the procedure of Laemmli, U.K. (1970) supra. The samples assayed are plasma and serum samples.

The samples are diluted 1/40 in distilled water. 100 µl of sample solution are boiled with 40 µl of 10X sample buffer for 20 min. The 140 µl sample is cooled to room temperature by convection and 60 µl of the cooled solution is applied to the gel.

The sample buffer has the following composition and includes the dye:

| | |
|---|---|
| Sample buffer (10X) 5.0ml | 2.5M Tris-HCl, pH 6.8 |
| 4.0ml | 2β-mercaptoethanol |
| 0.2ml | 0.5% (w/v) bromophenol blue |
| 0.8ml | Glycerol (88%, v/v) |
| 0.5g | Sucrose |
| 1.6g | SDS |

The samples are run at 45mA through the stacking gel and at 35mA through the separating gel until the dye front reaches the end of the gel.

The separated proteins are transferred onto nitrocellulose in a BioRad (BioRad is a Trade Mark) immunoblot apparatus at 70V for 3h. The transfer buffer used is Tris/glycine (pH 8.3) with 20% methanol.

A nitrocellulose membrane is blocked in 3% Blotto (3% Marvel (Marvel is a Trade Mark) dried milk in 50 mM Tris/HCl, pH 7.5, 150mM NaCl) for 1h. Constant agitation is maintained during this and subsequent incubations.

The nitrocellulose membrane is incubated in 1/100 dilution of antiserum overnight and washed five times for 10 min. each in TST (50 mM Tris/HCl, pH 7.5,150 mM NaCl, 0.05% Tween 20 (Tween is a Trade Mark). The membrane is blocked again for 1h. in 3% Blotto.

The membrane is incubated in 1/1000 dilution of $^{125}$I-Protein A (3 x $10^5$ cpm/ml) for 1h., followed by five washes as before.

The membrane is air dryed and exposed to x-ray film over 3-4 days at -70° C. The membrane is then warmed to 37° C and the film developed.

## EXAMPLE 8

### ELISA procedure for Δ-6 desaturase

A) Coating of the solid phase.

Polystyrene microtitre wells are coated with 150 μl of an appropriate dilution of the coating preparation e.g., a 1/16000 dilution of either polyclonal or monoclonal anti-Δ-6 desaturase antibody in carbonate coating buffer, pH 9.6.

The coated microtitre wells are incubated either overnight at 4° C or for 90 min. at 37° C.

The coated microtitre wells are then washed four times in washing buffer (See Example 7).

The coated microtitre wells may be used immediately or stabilised using a stabilising agent or stabilising method and stored until required. A suitable stabilising solution is 50% glycerol in 0.01 M phosphate buffered saline (PBS) / Bovine serum albumin (BSA) (1mg 1ml) solution.

B) Assay Procedure.

The stabilised niicrotitre wells are washed four times in washing buffer. A volume (e.g. 150 μl) of the sample to be assayed or an appropriate dilution thereof is added to the microtitre well.

Standards and controls (same volume as for the sample to be assayed) are added to a series of microtitre wells.

The samples, standards and controls are maintained for the required time i.e., overnight at 4° C or 60 min. at 37° C.

The microtitre wells are washed four times in washing buffer.

A volume (e.g. 100 μl) of anti-Δ-6 desaturase conjugate is added to the microtitre wells. A suitable conjugate is rabbit 1 gG - HRP conjugate prepared according to the maleimide method of Ishikawa, E. et al . (1983); Journal of Immunoassay 4(3), 209-327.

The conjugate containing wells are incubated for 60 min. at 37° C.

The microtitre wells are washed four times in washing buffer as before.

100 µl of OPD ( o -henylenediamine 2µg/ml) in 0.15M citrate-phosphate buffer, pH 5.0 are added to the microtitre wells, followed by incubation at room temperature for 30 min.. At the end of the incubation period the colour development is stopped by the addition of 50 µl of stopping solution; 4M $H_2SO_4$.

The optical density of the walls is read at 492nm. A standard curve is generated from the readings obtained from the standards in conventional manner e.g. by using a computer software package or manually. The analyte concentrations of the samples are calculated from the standard curve obtained.

## EXAMPLE 9

Western Blotting of various fractions of rat liver microsome with rabbit antisera prepared in Example 5.

Examples of the crude microsome fraction, solubilised fraction, fraction obtained from the DEAE-cellulose column and the fraction obtained from the CM-Sephadex column as described in Example 4 (see Scheme 4) were analysed by the Western Blotting technique (Towbin, H., Stachelin, H. and Gordon, J. (1979) Proceedings National Academy Sciences, U.S.A. 76, No. 9, pp 4350-4354), using appropriate molecular weight markers, on 12% SDS-PAGE gel by reacting the gel with rabbit antisera as prepared in Example 5.

It was found that the 66 K protein band reacts with the rabbit antisera. No band showed up in a control film using serum from a non-immunised rabbit as control.

## EXAMPLE 10

Western Blotting of human serum by anti-Δ-6 desaturase antibody

Human serum was run on SDS-PAGE gel with appropriate molecular weight markers, following which the gel was Western Blotted with anti-Δ-6 desaturase antibody. These experiments were carried out to show that the antisera produced to rat liver Δ-6 desaturase cross reacts with material in human sera. A crude microsomal fraction prepared from rat liver was used as a positive control to show that a reaction was obtained on Western Blotting of crude material. Concentrations of human serum used in the Western Blot ranged from 1 µg to 40 µg of serum protein applied to the gel. The crude microsomal fraction was used as a positive control. The results obtained showed that the anti-Δ-6 desaturase antibody reacts with a band in the human serum corresponding to a molecular weight in the range 66-67K.

No band showed up in a control film using serum from a non-immunized rabbit as control

## EXAMPLE 11

Δ-6 Desaturase inhibition assay

A Δ-6 desaturase inhibition assay was carried out by repeating the assay carried out in Example 2, but preincubating the rat liver microsomal preparation with IgG antisera obtained in Example 5 at a concentration of 4 mg IgG antisera/mg microsomal protein at 25°C for 10 min. The Relative Activity of Δ-6 desaturase for the microsome protein preparation so treated was found to be 68.4% which contrasted with a value of 90.05% for the microsome protein preparation preincubated with non-immunized rabbit IgG (4 mg IgG/mg microsome protein). Accordingly, the results showed that the enzyme is inhibited by the IgG antisera. This result is close to that obtained by Fujiwara, Y. et al. (1984) Archives of Biochemistry and Biophysics, Vol. 233, No. 2, September, pp. 402-407 in studies carried out to confirm the cytoplasmic location of Δ-6 desaturase in liver microsomes.

## Claims

1. A diagnostic method for the detection and determination of either component of the Δ-6 desaturase antigen-antibody reaction, which method comprises contacting a sample of a body fluid or secretion containing or suspected of containing the component to be detected with an amount of said other component and determining whether binding of said components has occurred.

2. An immunoassay method for the detection and determination of either component of the Δ-6 desaturase antigen-antibody reaction which comprises:

a) adding a sample of a body fluid or secretion containing or suspected of containing the component to be detected or determined to an amount of said other component of said Δ-6 desaturase antigen-antibody reaction in an insolubilised form;

b) allowing the immunochemical reaction to take place; and

c) determining the presence in said sample of said component by confirming the binding thereof to said insolubilised form of said one component as defined in step a).

3. An immunoassay method for the detection and determination of the Δ-6 desaturase antigen component of the Δ-6 desaturase antigen-antibody reaction which comprises:

a*) adding a sample of a body fluid or secretion containing or suspected of containing Δ-6 desaturase to an amount of anti-Δ-6 desaturase in an insolubilised form;

b*) allowing the immunochemical reaction to take place; and

c*) estimating the amount of Δ-6 desaturase bound to said insolubilised anti-Δ-6 desaturase.

4. An immunoassay method according to Claim 3, which comprises:

a**) adding a sample of a body fluid or secretion containing or suspected of containing Δ-6 desaturase to an amount of anti-Δ-6 desaturase in an insolubilised form;

b**) allowing the immunochemical reaction to take place, while simultaneously or subsequently adding a predetermined amount of labelled anti-Δ-6 desaturase; and

c**) estimating the amount of Δ-6 desaturase by determining the activity and/or amount of free or bound labelled anti-Δ-6 desaturase in a manner known per se.

5. An immunoassay method for the detection of anti-Δ-6 desaturase which comprises:

A) adding a sample of a body fluid or secretion containing or suspected of containing anti-Δ-6 desaturase to an amount of Δ-6 desaturase in an insolubilised form;

B) allowing the immunochemical reaction to take place; and

C) determining the presence in said sample of anti-Δ-6 desaturase by confrrming the binding thereof to said insolubilised Δ-6 desaturase.

6. A method according to any one of Claims 1-4 for determining levels of Δ-6 desaturase throughout the menstrual cycle in females indicative of a predisposition to, or confirming the likelihood of, premenstrual syndrome in such females and which can be alleviated by a source of gamma-linolenic acid.

7. A method according to any one of Claims 1-6, wherein the body fluid is blood or a blood fraction or urine.

8. A test kit containing one or more component(s) for carrying out a method according to any one of Claims 1-7.

9. A test kit for the detection and determination of Δ-6 desaturase in a body fluid or secretion by immunoassay which comprises:

A′) a given quantity of insolubilised anti-Δ-6 desaturase or the necessary ingredients for preparing said insolubilised anti-Δ-6 desaturase; and

B′) a given quantity of the coupling product of a label with anti-Δ-6 desaturase.

10. A method for the preparation of polyclonal antibody for use in a method according to any one of Claims 1-9, which comprises immunising a mammalian host with Δ-6 desaturase bound to a carrier by injection intradermally or intraperitoneally, bleeding said host and collecting the antiserum.

European Patent Office

# EUROPEAN SEARCH REPORT

Application Number

EP 90 30 7857

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 111, no. 25, 18th December 1989, page 309, abstract no. 227640t, Columbus, Ohio, US; A. SCHMIDT et al.: "Immunological detection of phytoene desaturase in algae and higher plants using an antiserum raised against a bacterial fusion-gene construct", & EUR. J. BIOCHEM., 1989, 184(2), 375-8 * The entire abstract * --- | 1 | G 01 N 33/573<br>G 01 N 33/543<br>C 07 K 3/02 |
| Y | THE JOURNAL OF BIOLOGICAL CHEMISTRY, vol. 255, no. 6, 25th March 1980, pages 2583-2589, Baltimore, US; M.R. PRASAD et al.: "Topology of the delta9 terminal desaturase in chicken liver microsomes and artificial micelles" * Pages 2583-2584 * --- | 1-10 | |
| Y | GB-A-2 078 953 (INTERNATIONAL IMMUNOASSAY LABORATORIES, INC.) * Abstract; page 1, lines 1-51 * --- | 1-10 | **TECHNICAL FIELDS SEARCHED (Int. Cl.5)** |
| A | BIOLOGICAL ABSTRACTS, vol. 79, no. 7, 1985, page AB-329, columns 1-2, abstract no. 57942, Biological Abstracts, Inc., Philadelphia, PA, US; Y. FUJIHARA et al.: "Immunological specifity and cytoplasmic location of delta6-desaturase in microsomal membrane", & HOKKAIDO J. MED. SCI., 59(4): 446-456 --- -/- | | G 01 N |

The present search report has been drawn up for all claims

| Place of search | Date of completion of the search | Examiner |
|---|---|---|
| THE HAGUE | 09-10-1990 | GRIFFITH G. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 90 30 7857

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | BIOLOGICAL ABSTRACTS, vol. 76, no. 4, 1983, page 2768, column 2, abstract no. 25563, Biological Abstracts, Inc., Philadelphia, PA, US; Y. FUJIWARA et al.: "Immunochemical of evidence for the enzymatic difference of delta6-desaturase from delta9-desaturase in rat liver microsomes", & BIOCHEM. BIOPHYS. RES. COMMUN., 110(1): 36-41<br>--- | | |
| A | BIOLOGICAL ABSTRACTS, vol. 64, no. 6, 1977, page 3166, column 2 - page 3167, column 1, abstract no. 32245, Biological Abstracts, Inc., Philadelphia, PA, US; I. JANSSON et al.: "Studies on the three microsomal electron transfer enzyme systems: Specifity of electron flow pathways", & ARCH. BIOCHEM. BIOPHYS., 178(1): 89-107, 1977<br>----- | | TECHNICAL FIELDS SEARCHED (Int. Cl.5) |

The present search report has been drawn up for all claims

| Place of search | Date of completion of the search | Examiner |
|---|---|---|
| THE HAGUE | 09-10-1990 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)